# EUROPEAN PATENT APPLICATION

(11) **EP 4 570 220 A1**
(43) Date of publication of application: **18.06.2025**
(21) Application number: 23851131.5
(22) Date of filing: 10.08.2023
(51) Int. Cl.: A61F 13/02, A61M 1/00

(54) **DRESSING FOR NEGATIVE PRESSURE THERAPY, DEVICE FOR MANUALLY GENERATING AND INDICATING NEGATIVE PRESSURE, A KIT, A MANUFACTURING PROCESS AND NEGATIVE PRESSURE THERAPY**

(30) Priority: 10.08.2022 BR 102022015836
(71) Applicant: Blue Hands Serviços Médicos S/S, 01308000 São Paulo (BR)
(72) Inventor: OKSMAN, Denis, 04565-001 São Paulo (BR); ARRUDA, Rodrigo Garcia De, 04079-010 São Paulo (BR)
(74) Representative: Pace Napoleone, Maria
(86) International application number: PCT/BR2023/050258
(87) International publication number: WO 2024/031165

(57) **Abstract**

The present invention is in the fields of mechanical engineering and medicine, focused on the area of dressings and devices for negative pressure therapies. More specifically, the invention discloses an improved dressing, a device that provides the generation and indication of negative pressure that can be detachably connected to a dressing, a kit, a manufacturing process and a negative pressure therapy. The dressing of the invention comprises a valved connector detachably connectable to a device for manual generation of negative pressure and optionally comprises a means for colorimetric indication of negative pressure. The device of the invention provides manual generation of negative pressure and also visually indicates the negative pressure, for the user to verify that the negative pressure is within a range suitable for treatment, which preferably includes alternating the intensity of the negative pressure. The invention provides several practical advantages, including: the generation and indication of negative pressure in a simplified manner; the reduction of costs when compared with the costs of electronic devices for indicating negative pressure; the expansion of access to patients to the benefits of negative pressure therapy; use in non-hospital conditions.

## Description

### Field of the Invention

The present invention is in the fields of mechanical engineering and medicine, focused on the area of dressings and devices for negative pressure therapies. More specifically, the invention discloses an improved dressing, a device that provides the generation and indication of negative pressure that can be detachably connected to a dressing, a kit, a manufacturing process and a negative pressure therapy. The dressing of the invention comprises a valved connector detachably connectable to a device for manual generation of negative pressure and optionally comprises a means for colorimetric indication of negative pressure. The device of the invention provides for the manual generation of negative pressure and also visually indicates the negative pressure, for the user to verify whether the negative pressure is within a range suitable for treatment, which preferably includes alternating the intensity of the negative pressure. The invention provides several practical advantages, including: the generation and indication of negative pressure in a simplified manner; the reduction of costs when compared with the costs of electronic devices for indicating negative pressure; the expansion of access to patients to the benefits of negative pressure therapy; use in non-hospital conditions.

### Background of the Invention

Negative pressure therapies have gained prominence in recent years due to their wide-ranging benefits, with dressings that work under negative pressure and devices that generate negative pressure within said dressings becoming available. Negative pressure therapies are predominantly used for wound treatment, but are not limited to that context.

The treatment of open or chronic wounds that are too complex to close spontaneously is well known in the art. Wound treatments using negative pressure wound therapy (NPWT) currently known in the art involve placing a dressing with a fluid-impermeable or semi-permeable film over the wound, using different means, such as adhesive films, to seal and isolate the dressing film around the wound, and connecting a negative pressure generating source, usually a vacuum pump, to the dressing, so that negative pressure is created and maintained under the dressing, that is, creating an environment with negative pressure between the wound and the dressing film.

Negative pressure wound therapy (NPWT) promotes healing of wounds such as burns, hypertrophic scar resections, exposed fractures, extensive soft tissue injuries, bedsores, surgical wound dehiscence, etc., facilitating the formation of granulation tissue at the wound site and supporting the body's normal inflammatory process, while simultaneously removing excess exudate, which may contain cytokines and/or adverse bacteria.

In this sense, as mentioned above, dressings used in negative pressure wound therapy (NPWT) use a device equipped with a motor/pump that remains connected to the dressing during use, in which the device, when activated, promotes a "vacuum", with pressure below atmospheric pressure - negative or subatmospheric pressure - between the wound and the dressing. This negative pressure applied to the wound region then optimizes wound healing.

However, the high cost of these dressings with negative pressure therapy devices is a limiting factor for large-scale use, so that access to the benefits of using negative pressure therapy to improve wound healing is restricted to a small portion of the population that can afford to purchase the device.

The low usability of the dressing + vacuum generating device is also a limiting factor for the use of negative pressure therapy in wound healing. On the one hand, in the conventional approach the user needs to carry a vacuum generating pump connected to the dressing while using the dressing, which makes it uncomfortable and impractical to use.

Furthermore, these state of the art dressings + vacuum generating devices do not have intuitive and practical usability, so that the user often needs the assistance of a health professional to change the dressing and/or to handle the device, forcing the user to go to a medical center or request home care to assist him/her.

In the search for the state of the art in scientific and patent literature, the following documents were found that deal with the topic:
US11497653B2 discloses a dressing set and vacuum generating device, commercially known by the name "PICO^{™} Dressing", in which the dressing has a channel that connects to the vacuum generating device, so that the device remains connected to the dressing and, through a controller, it maintains the negative pressure at a constant and predetermined value throughout use. Furthermore, PICO^{™} is used essentially to treat open wounds. In this way, the dressing + device set disclosed by US11497653B2 requires the user to carry the vacuum generating device connected to the dressing with him/her during the time he/she is using the dressing, which makes it uncomfortable and difficult to use, since the wound may be located in a part of the body that is difficult to access, which makes it very difficult to carry the device near the wound.

US2020289726A1 discloses another dressing and vacuum generating device set, wherein the dressing has a channel that connects to the vacuum generating device and a pressure indicator disposed on top of the dressing that indicates changes in pressure within the dressing. Said indicator may be a fluid conductor, a pneumatic spring configured to move when subjected to an increase in pressure, a compressible foam, a molded polymer, and holograms or liquid crystal displays. Furthermore, the vacuum generating device also remains connected to the dressing and, through a controller, maintains the negative pressure at a predetermined value throughout use. In this way, the dressing + device set disclosed by US2020289726A1 requires the user to carry the vacuum generating device connected to the dressing with him/her during the time he/she is using the dressing, which makes use uncomfortable and difficult, since the wound may be located in a part of the body that is difficult to access, which makes carrying the device near the wound very uncomfortable and unpleasant.

US2012071845A1 discloses a device that generates and maintains negative pressure in the dressing and suctions wound exudate into the dressing, the device being commercially known as the "SNAP^{™} Therapy Cartridge". To this end, the device has a suction chamber that stores the exudate sucked from the wound and sensors that detect whether the suction chamber's storage capacity is exhausted, and the device can be configured to generate and substantially maintain negative pressure in the dressing at a fixed and predetermined value. Furthermore, SNAP^{™} is used primarily to treat open wounds, as it has a suction chamber that stores the exudate eliminated from open wounds and cannot be used to treat closed wounds. Thus, the device disclosed in document US2012071845A1 remains connected to the dressing to suck the exudate and to maintain the negative pressure at the predetermined value, i.e., it forces the user to carry the device connected to the dressing with him/her while he/she is using the dressing, which makes it uncomfortable and difficult to use, since the wound may be located in a part of the body that is difficult to access, compromising the flexibility of application to wounds on any part of the body.

In this sense, the state of the art negative pressure generating devices, in addition to being complex, require a permanent connection to the dressing to enable detection of the negative pressure value inside the dressing and to maintain the negative pressure at a predetermined value. These limitations bring several disadvantages to their applications and user usability, both due to complexity and cost, limiting access to this type of therapeutic approach, and due to the inconvenience of the user needing to carry the device with him/her while using the dressing.

State of the art devices have a high acquisition cost and only work during the period of use of the dressing, generating the need to acquire a new device when the wound dressing is changed by the user or the medical team. This limits access to the benefits of using negative pressure therapy to a small portion of the population with the financial resources to purchase multiple devices during wound treatment.

US20090259203A1 discloses a dressing with a modular structure that allows its length to be changed according to the size and shape of the wound. Said dressing is equipped with a suction device having a spring and a sliding mechanism and a one-way valve, so that the mechanism can be pulled back to create negative pressure or maintain a constant level of pressure in the dressing. Furthermore, the dressing has another configuration where it has a device equipped with a spring, where the spring creates a mobile seal through a piston that self-regulates the pressure changes in the dressing. However, the dressing disclosed in US20090259203A1 is uncomfortable to use, since the mechanisms that self-regulate the negative pressure in the dressing are fixed to the dressing itself, compromising the malleability of the dressing and the flexibility of application to wounds anywhere on the body.

US2021169699A1, similarly to US20090259203A1, discloses a dressing having a vacuum pump with a one-way valve connected on top of the dressing, so that the vacuum pump can be manually squeezed/compressed to create negative pressure in the dressing. However, the dressing disclosed in US2021169699A1 is uncomfortable to use, since the vacuum pump that generates negative pressure in the dressing is fixed to the dressing itself, compromising the malleability of the dressing and the flexibility of application to wounds anywhere on the body.

The dressings used in state of the art negative pressure therapy have negative pressure generating devices/pumps arranged next to the dressing, which negatively affects the user's usability and generates discomfort and inconvenience to the user due to using a dressing with rigid structures on the upper part. These limitations impair the flexibility of applying the dressing to wounds anywhere on the body, as well as affecting the malleability of the dressing and, consequently, its comfort when applied to the user's wound.

From what can be inferred from the literature researched, no documents were found anticipating or suggesting the teachings of the present invention, so that the solution proposed here has novelty and inventive activity compared to the state of the art.

In this sense, the state of the art lacks dressings useful in negative pressure therapy and/or negative pressure generating devices that provide portability, flexibility and easy access for the user to perform the therapy under medical guidance, and/or that provide easy connection and/or disconnection of the dressing from the negative pressure generating device.

### Summary of the Invention

The present invention solves the problems of the state of the art based on an inventive concept that provides, simultaneously and without the need for batteries or electrical energy sources, the manual generation of negative pressure in a dressing through a removable connectable device and the indication of the negative pressure.

An important aspect of the invention is to provide negative pressure therapy with alternating negative pressure intensity. Alternating negative pressure intensity is clinically relevant and has been shown to be more efficient than continuously maintaining a fixed level of negative pressure. In the present invention, this technical effect of alternating negative pressure intensity is achieved by:
- placing the dressing of the invention on the skin;
- manually generating negative pressure in said dressing until a negative pressure limit is reached, indicated by the visual indicator; and
- one or more new steps of manual generation of negative pressure when the negative pressure is close to or below a minimum negative pressure limit.

Among other advantages, the present invention provides greater access for patients to the benefits of negative pressure therapy, through the manual generation of negative pressure and the indication of negative pressure mechanically or colorimetrically, reducing costs when compared to the high costs of electronic devices for generating and indicating negative pressure.

In a first object, the present invention provides a negative pressure therapy dressing comprising:
- a modular structure having an adhesive film connected around a sealing film positioned above at least one absorbent layer; and
- at least one valved connector connected to the sealing film, wherein the valved connector is detachably connectable to at least one device for generating negative pressure.

In one embodiment, the modular structure of said dressing additionally comprises a colorimetric negative pressure indicator and the negative pressure is generated by a conventional hypodermic syringe.

In one embodiment, the dressing has a geometry in the shape of a planar dressing for placement over the skin. In another embodiment, the dressing has a geometry in the shape of or similar to an Unna Boot, being sealed at the height of the leg and at the end of the feet before the toes.

The invention also provides a device for manually generating negative pressure in a dressing, which also indicates the negative pressure generated in the dressing.

In one embodiment, the manual generation of negative pressure and its indication is done through two interconnected body-plunger sets, the first being removably connectable to the dressing to manually generate negative pressure therein, and the second connected to the body of the first and comprising a negative pressure indication mechanism. In this embodiment, manual actuation of the first plunger creates negative pressure in the dressing and also causes the second plunger, which indicates the negative pressure in the dressing, to move. The pressure difference generated (between the negative pressure inside the dressing and the external atmospheric pressure) is indicated to the user, who can then check whether the negative pressure in the dressing is within a range suitable for treating the wound.

In a second object, therefore, the present invention presents a device useful for negative pressure therapy, said device comprising at least two body-plunger sets:
- the first containing a body that can be detachably connected to a dressing to which negative pressure is to be created, and at least one first manually operable plunger that can be slidably arranged in an internal region of the body; and
- the second containing a body fixedly connected to the body of the first plunger through at least one channel communicating with the internal region of the body and at least a second plunger provided with a negative pressure indication mechanism arranged internally to the channel,
the value of the negative pressure being indicated through at least one visual indicator according to the movement of the second plunger indication mechanism, caused by the manual activation of the first plunger.

In one embodiment, said body-plunger sets are hypodermic syringes.

In a third object, the present invention presents a manufacturing process for a negative pressure generation and indication device comprising the steps of:
- forming a fluidic connection point in the body of a body-plunger set containing a body removably connectable to a dressing to which negative pressure is desired, and at least one first manually operable plunger slidably disposed in an internal region of the body; and
- connecting said fluidic connection point to the body of a second body-plunger set through at least one channel communicating with the internal region of the first body, the second body- plunger set comprising at least a second plunger provided with a negative pressure indication mechanism arranged internally to the channel of the second body.

In a fourth object, the invention presents a kit for negative pressure therapy comprising:
- a dressing comprising a modular structure provided with an adhesive film connected around a sealing film positioned above at least one absorbent layer and at least one valved connector connected to the sealing film, wherein the valved connector is detachably connectable to at least one device for generating negative pressure; and
- a device comprising at least two body-plunger sets, the first containing a body removably connectable to a dressing to which negative pressure is desired, and at least one manually operable plunger slidably disposed in an internal region of the body, and
- the second containing a body fixedly connected to the body of the first plunger through at least one channel communicating with the internal region of the body and at least a second plunger provided with a negative pressure indication mechanism arranged internally to the channel.

In a fifth object, the present invention presents a negative pressure therapy comprising:
- placing the dressing of the invention on the skin;
- manually generating negative pressure in said dressing until a negative pressure limit is reached, indicated by the visual indicator; and
- one or more new steps of manual generation of negative pressure when the negative pressure is close to or below a minimum negative pressure.

In one embodiment, said therapy is a method for treating incisional wounds using negative pressure therapy comprising the steps of:
a. applying at least one dressing to the wound on the user's skin, wherein the dressing comprises an adhesive film connected around a sealing film, wherein the sealing film is positioned above at least one absorbent layer and connected to at least one valved connector;
b. isolating the internal environment of the dressing by adhesive film to the user's skin;
c. detachable connecting a negative pressure generation and indication device to the valved connector;
d. generating negative pressure in the internal environment of the dressing using the negative pressure generation and indication device;
e. indicating the range of negative pressure values to which the internal environment of the dressing is subjected through the negative pressure generation and indication device; and
f. disconnecting the negative pressure generation and indication device from the valved connector.

In another embodiment, said therapy is a cosmeceutical method for treating skin using negative pressure therapy comprising the steps of:
a. applying at least one dressing to the user's skin, wherein the dressing comprises an adhesive film connected around a sealing film, wherein the sealing film is positioned above at least one absorbent layer and connected to at least one valved connector;
b. isolating the internal environment of the dressing by adhesive film to the user's skin;
c. detachable connecting a negative pressure generation and indication device to the valved connector;
d. generating negative pressure in the internal environment of the dressing using the negative pressure generation and indication device;
e. indicating the range of negative pressure values to which the internal environment of the dressing is subjected through the negative pressure generation and indication device; and
f. disconnecting the negative pressure generation and indication device from the valved connector.

These and other objects of the invention will be immediately appreciated by those skilled in the art and will be described in detail below.

### Brief Description of the Figures

The following figures are presented:
Fig. 1 shows an embodiment of the negative pressure generation and indication device (5) of the present invention, illustrating the first plunger (1) fully inserted into the body (12), as well as illustrating the channel (13) of the second plunger (8) comprising an elastic element (4) which moves an indication mechanism (2) according to the negative pressure measured within the internal environment of the dressing (30), indicating the range of values of said negative pressure through the visual indicator (3).
Fig. 2 shows an embodiment of the negative pressure generation and indication device (5) of the present invention, illustrating the first plunger (1) pulled in relation to the body (12), forming a first environment at the first end (10) of the channel (13) with a hollow flap (1.2) that receives the air coming from the internal environment of the dressing (30) and a second environment at the second end (11) of the channel (13) that is hollow for the entry of atmospheric air.
Fig. 3 shows a side view of an embodiment of the negative pressure generation and indication device (5) of the present invention, illustrating a state in which the elastic element (4) is in a contracted position, which has moved the indication mechanism (2) into a position that indicates, through the visual indicator (3), that the pressure range detected in the internal environment of the dressing (30) is inadequate (red band) for treatment with negative pressure therapy.
Fig. 4 shows another side view of an embodiment of the negative pressure generation and indication device (5) of the present invention, illustrating a state in which the first plunger (1) has been pulled and the elastic element (4) is in a distended position, which has moved the indication mechanism (2) into a position that indicates, through the visual indicator (3), that the pressure range detected in the internal environment of the dressing (30) is adequate (green band) for treatment with negative pressure therapy.
Fig. 5 shows a cross-sectional side view of an embodiment of the negative pressure generation and indication device (5) of the present invention, illustrating in detail the first plunger (1) inserted in the body (12), as well as the second end (11) of the channel (13) hollowed out the atmosphere.
Fig. 6 shows an embodiment of the first plunger (1) of the device (5) of the present invention, illustrating in detail the flap (1.2) hollowed out to receive air from the inside of the dressing (30) and the second end (11) of the channel (13) hollowed out to receive atmospheric air.
Fig. 7 shows an embodiment of the body (12) of the device (5) of the present invention, illustrating in detail the nozzle (7) with threaded geometry.
Fig. 8 shows an embodiment of the shape of the holes in the flap (1.2) of the first end (10) of the device (5).
Fig. 9 shows another embodiment of the shape of the holes in the flap (1.2) of the first end (10) of the device (5).
Fig. 10 shows an embodiment of the dressing (30) of the present invention, illustrating the valved connector (26) connected to the sealing film (20) and with a colorimetric indicator (24) arranged inside the valved connector (26).
Fig. 11 shows another embodiment of the dressing (30) of the present invention, illustrating the valved connector (26) connected to the sealing film (20) and with a colorimetric indicator (24) arranged between the sealing film (20) and the absorbent layer (22).
Fig. 12 shows another embodiment of the dressing (30) of the present invention, illustrating the valved connector (26) connected to the sealing film (20).
Fig. 13 shows another embodiment of the dressing (30) of the present invention, illustrating the valved connector (26) connected to a hose (23) connected to the sealing film (20) and with a colorimetric indicator (24) arranged inside the valved connector (26).
Fig. 14 shows another embodiment of the dressing (30) of the present invention, illustrating the valved connector (26) connected to a hose (23) connected to the sealing film (20) and with a colorimetric indicator (24) arranged between the sealing film (20) and the absorbent layer (22).
Fig. 15 shows another embodiment of the dressing (30) of the present invention, illustrating the valved connector (26) connected to a hose (23) connected to the sealing film (20).
Fig. 16 shows a developed prototype of the negative pressure generation and indication device (5) of the present invention, illustrating the pulling movement of the first plunger (1) and the consequent contraction movement of the indication mechanism (2) of the second plunger (8).
Fig. 17 shows a developed prototype of the negative pressure generation and indication device (5) of the present invention, illustrating the first plunger (1) inserted into the body (12) and the consequent distension movement of the indication mechanism (2) of the second plunger (8).
Fig. 18 shows another developed prototype of the negative pressure generation and indication device (5) of the present invention, illustrating the second plunger (8) arranged internally to the first plunger (1) and the body (12).

### Detailed Description of the Invention

In the context of the present invention, the expression "negative pressure therapy" refers to therapy with negative pressure on the skin (6) and includes medical therapies, such as the treatment of incisional wounds, and also other therapies on the skin (6) without involving incisions, such as wounds resulting from infections, or merely for the modulation of the physiological state of the skin (6), whether by therapeutic, cosmetic or cosmeceutical approach.

Among other advantages, the present invention provides greater access for patients/users to the benefits of negative pressure therapy, through the manual generation of negative pressure and the indication of negative pressure mechanically or colorimetrically, reducing costs when compared to the high costs of electronic negative pressure formation and indication devices.

The present invention provides an approach that enables improved negative pressure therapy comprising: placing the dressing of the invention on the skin (6); manually generating negative pressure in said dressing (30) until a negative pressure limit is reached, indicated by the visual indicator (3); and one or more new steps of manually generating negative pressure when the negative pressure is close to or below a minimum negative pressure limit.

The inventive concept common to the various objects of the invention is to provide, concomitantly and without the need for batteries or electrical energy sources, the manual generation of negative pressure in a dressing (30) through a removable connectable device (5) and the indication of the negative pressure.

The present invention provides a dressing (30) useful for negative pressure therapy, a device (25) for manually generating negative pressure in said dressing, and a kit comprising the dressing (30) and the device (5). The dressing (30) or the device (5) comprises a pressure indicator to indicate to the user whether the range in which the negative pressure in the dressing is within a predetermined range. A manufacturing process and a therapeutic method or negative pressure therapy are also disclosed.

In a first object, the present invention provides a dressing for negative pressure therapy comprising:
- a modular structure provided with an adhesive film (21) connected around a sealing film (20) positioned above at least one absorbent layer (22); and
- at least one valved connector (26) connected to the sealing film (22), wherein the valved connector (26) is detachably connectable to at least one device for generating negative pressure (5).

In one embodiment, the modular structure of said dressing (30) additionally comprises a colorimetric indicator (24) of negative pressure and the negative pressure is generated by a conventional hypodermic syringe.

In a second object, the present invention presents a device useful for negative pressure therapy, said device comprising at least two body-plunger sets:
- the first containing a body (12) detachably connectable to a dressing (30) to which negative pressure is desired, and at least one first plunger (1) manually operable and arranged in a sliding manner in an internal region of the body (12); and
- the second containing a body fixedly connected to the body of the first plunger (1) through at least one channel (13) communicating with the internal region of the body (12) and at least one second plunger (8) equipped with an indication mechanism (2) of negative pressure arranged internally to the channel (13), the value of the negative pressure being indicated through at least one visual indicator (3) according to the movement of the indication mechanism (2) of the second plunger (8), caused by the manual activation of the first plunger (1).

In one embodiment, said body-plunger sets are hypodermic syringes.

In a third object, the present invention presents a manufacturing process for a negative pressure generation and indication device comprising the steps of:
- forming a fluidic connection point on the body of a body-plunger set containing a body (12) removably connectable to a dressing (30) to which negative pressure is desired, and at least one first plunger (1) manually operable and slidably arranged in an internal region of the body (12); and
- connecting said fluidic connection point to the body (12) of a second body-plunger set through at least one channel (13) communicating with the internal region of the first body (12), the second body-plunger set comprising at least one second plunger (8) provided with an indication mechanism (2) of negative pressure arranged internally to the channel (13) of the second plunger (8).

In a fourth object, the invention presents a kit for negative pressure therapy comprising:
- a dressing (30) comprising a modular structure provided with an adhesive film (21) connected around a sealing film (20) positioned above at least one absorbent layer (22) and at least one valved connector (26) connected to the sealing film (20), wherein the valved connector (26) is detachably connectable to at least one device for generating negative pressure (5); and
- a device (5) comprising at least two body-plunger sets, the first containing a body (12) removably connectable to a dressing (30) to which negative pressure is desired, and at least one first plunger (1) manually operable and slidably arranged in an internal region of the body (12), and
- the second containing a body fixedly connected to the body of the first plunger (1) through at least one channel (13) communicating with the internal region of the body (12) and at least a second plunger (8) equipped with an indication mechanism (2) of negative pressure arranged internally to the channel (13).

In a fifth object, the present invention presents a negative pressure therapy comprising:
- placing the dressing (30) of the invention on the skin (6);
- the manual generation of negative pressure in said dressing (30) until a negative pressure limit is obtained, indicated by the visual indicator (3); and
- one or more new steps for manually generating negative pressure when the negative pressure is close to or below a negative pressure minimum limit.

In one embodiment, said therapy is a method for treating incisional wounds using negative pressure therapy comprising the steps of:
a. applying at least one dressing (30) to the wound on the user's skin (6), wherein the dressing (30) comprises an adhesive film (21) connected around a sealing film (20), wherein the sealing film (20) is positioned above at least one absorbent layer (22) and connected to at least one valved connector (26);
b. isolating the internal environment of the dressing (30) by adhesiveness of adhesive film (21) to the user's skin (6);
c. detachably connecting a negative pressure generation and indication device (5) to the valved connector (26);
d. generating negative pressure in the internal environment of the dressing (30) D the negative pressure generation and indication device (5);
e. indicating the range of negative pressure values to which the internal environment of the dressing (30) is subjected by means of the negative pressure generation and indication device (5); and
f. disconnecting the negative pressure generation and indication device (5) from the valved connector (26).

In another embodiment, said therapy is a cosmeceutical method for treating skin (6) using negative pressure therapy comprising the steps of:
a. applying at least one dressing (30) on the skin (6) of the user, wherein the dressing (30) comprises an adhesive film (21) connected around a sealing film (20), wherein the sealing film (20) is positioned above at least one absorbent layer (22) and connected to at least one valved connector (26);
b. isolating the internal environment of the dressing (30) by adhesiveness of adhesive film (21) to the user's skin (6);
c. detachably connecting a negative pressure generation and indication device (5) to the valved connector (26);
d. generating negative pressure in the internal environment of the dressing (30) by means of the negative pressure generation and indication device (5);
e. indicating the range of negative pressure values to which the internal environment of the dressing (30) is subjected by means of the negative pressure generation and indication device (5); and
f. disconnecting the negative pressure generation and indication device (5) from the valved connector (26).

Embodiments of the objects of the invention are described in more detail below.

An embodiment of the negative pressure generating and indicating device for negative pressure therapy comprises at least one body (12) and at least one first plunger (1) slidably arranged in an internal region of the body (12), wherein the first plunger (1) comprises:
- at least one channel (13) communicating with the internal region of the first plunger (1) that extends along the length of the first plunger (1);
- at least one indication mechanism (2) arranged internally to the channel (13); and
- at least one visual indicator (3) associated with the channel (13);
wherein the indication mechanism (2) moves along the inner part of the channel (13) to measure a negative pressure value, the value being indicated through the visual indicator (3).

In one embodiment, the second plunger is arranged in the inner part of the channel (13), which is arranged along the entire length of the second plunger (8), i.e., the second plunger (8) is hollow along its length, forming the channel (13). In another embodiment, the channel (23) is positioned externally to the second plunger (8), the channel (23) being communicating with the plunger (1). In another embodiment, the channel (13) is positioned externally to the second plunger (8) and being arranged parallel to the length of the first plunger (1).

In one embodiment, the channel of the second plunger (8) comprises two environments separated and isolated by the indication mechanism (2), the environments being: a first environment comprising at least a first end (10) with a flap (1.2) hollow for the air intake from the internal environment of the dressing and a second environment comprising at least a second end (11) hollow for the intake of atmospheric air.

In one embodiment, the indication mechanism (2) comprises an isolation means that isolates the first environment from the second environment, comprising at least one material among: rubber; polymer; or a combination thereof.

In one embodiment, the indicating mechanism (2) comprises at least one elastic element (4). In one embodiment, the elastic element (4) comprises at least one of: sponge; polymer; spring; pneumatic spring; or a combination thereof.

In one embodiment, the elastic element (4) is fixed to the first end (10) of the first environment of the channel (13) by means of at least one flap (1.2), so that the spring (4) extends along the length of the channel (13) from the flap (1.2).

In this way, the elastic element (4) extends and contracts along the second environment of the channel (13), displacing the indication mechanism (2).

In one embodiment, the visual indicator (3) is arranged parallel to the length of the channel (13), i.e., along the length of the first and second environments, and comprises at least one of: a colorimetric indicator; a numerical indicator; a geometric indicator; or a combination thereof. In one embodiment, the visual indicator (3) comprises a negative pressure indication range comprising micro indications, which indicate more specific ranges of values within the range of the visual indicator (3) according to the therapy indicated for each type of application.

In this way, the indication mechanism (2) moves along the channel (13) through the distention and contraction of the elastic element (4) and indicates, by means of the visual indicator (3), the negative pressure range measured in the internal environment of the dressing (30).

Thus, when the negative pressure generation and indication device (5) is connected to the dressing (30), the elastic element (4) moves along the second environment of the channel (13), displacing the indication mechanism (2) and indicating the negative pressure range measured within the dressing (30), guiding the user as to whether or not to generate more negative pressure, depending on the indication shown.

In one embodiment, the first plunger (1) additionally comprises at least one pulling handle (9) with geometry adapted to fit the user's fingers and assist him/her in the action of pulling the first plunger (1) to suck the air from the inside of the dressing, promoting the generation of negative pressure in the inside of the dressing (30). In this way, the pulling handle (9) promotes greater accessibility/usability by allowing the user to pull the first plunger (1) using only one hand.

In one embodiment, the body (12) of the negative pressure generation and indication device (5) comprises at least one nozzle with threaded geometry (7) connectable to at least one closed-system valved connector (26) connected to the dressing (30). In one embodiment, the nozzle (7) is a Luer-Lock type nozzle.

In this sense, the negative pressure generation and indication device (5) of the present invention is devoid of any electrical/electronic element, so that it generates and indicates negative pressure in a purely manual/mechanical manner, without the use of a battery or any form of electrical power supply, unlike the negative pressure/vacuum generation and indication devices of the prior art that use electronic and electrical components to generate, maintain and indicate negative pressure in the dressing.

Thus, this negative pressure generation and indication device gives several benefits to the user by providing greater comfort and ease of use due to the easy connection and/or disconnection of the device from the dressing when it is not in use. The device also provides benefits to the manufacturer due to its simplicity and low manufacturing cost due to its geometry and easy-to-produce components, enabling large-scale production at low costs to expand access to this type of therapy.

In one embodiment, the process of manufacturing a negative pressure generation and indication device for negative pressure therapy comprises the steps of:
- manufacturing at least one body (12) comprising at least one nozzle with threaded geometry (7);
- manufacturing at least one plunger (1) comprising the steps of:
   a. opening an access to communicate at least one channel (13) to the internal region of the first plunger (1), wherein the channel (13) extends along the length of the first plunger (1);
   b. arranging at least one indication mechanism (2) internally to the channel (13); and
   c. arranging at least one visual indicator (3) associated with the channel (13);
- fitting the first plunger (1) in a sliding manner into the internal region of the body (12).

In one embodiment of said process, the opening of the channel (13) is manufactured in such a way as to comprise two environments separated and isolated by the indication mechanism (2), being: a first environment comprising at least a first end (10) with at least one flap (1.2) hollow for the air intake from the internal environment of the dressing (30); and a second environment comprising at least a second end (11) hollow for the intake of atmospheric air.

In one embodiment, the manufacturing process comprises an additional step of attaching the indication mechanism (2) to at least one elastic element (4), wherein the elastic element (4) is attached to the first end (10) of the inner part of the channel (13) through at least one flap (1.2), so that the elastic element (4) extends along the length of the channel (13).

In one embodiment, the indication mechanism (2) comprises an isolation means that isolates the first environment from the second environment and is manufactured with at least one material among: rubber; polymer; or a combination thereof.

In one embodiment, the visual indicator (3) is arranged parallel to the length of the channel (13), i.e., along the length of the first and second environments.

In one embodiment, the body (12) and the first plunger (1) are manufactured from transparent material to allow visualization of the displacement of the indication mechanism (2) and, consequently, visualization of the indication corresponding to the negative pressure generated in the dressing through the visual indicator (3).

In one embodiment, the manufacturing process of the device of the present invention comprises an additional step of manufacturing at least one pulling handle (9) connected to the end of the first plunger (1).

In this sense, said pulling handle (9) comprises geometry adapted to fit the user's fingers and assist him/her in pulling the first plunger (1), so that, when the device is connected to the dressing, the action of pulling the first plunger (1) sucks the air from the inner part of the dressing (30) and promotes the generation of negative pressure in the inner part of the dressing (30). In this way, the pulling handle (9) promotes greater accessibility/usability by allowing the user to pull the first plunger (1) using only one hand.

Furthermore, in one embodiment, the body (12) and the first plunger (1) are manufactured from disposable polymeric material, which allows for safe, sustainable disposal and simple recycling of the device, and prevents reuse of the contaminated device.

In one embodiment, the size of the negative pressure generation and indication device (5) varies according to the application, size of the skin to be treated and/or complexity of the wound which, more specifically, varies according to the size of the dressing used on the wound.

In this sense, the negative pressure generation and indication device (5) of the present invention comprises a simple manufacturing structure and allows the indication of negative pressure mechanically, not involving any electronic component, reducing manufacturing and marketing costs when compared with the high costs of electronic negative pressure indication devices of the state of the art, optimizing production and allowing greater access of patients to the benefits of negative pressure therapy.

One of the features of the invention is to provide easy and quick disconnection of the device for manual generation of pressure to the dressing when the device is not in use, which provides greater comfort in usability for the user, since the user does not need to carry the device with him/her connected to the dressing. The volume is also substantially reduced, enabling use in visible and/or sensitive areas.

In one embodiment, the dressing (30) for negative pressure therapy comprises a modular structure provided with an adhesive film (21) connected around a sealing film (20), wherein the sealing film (20) is positioned above at least one absorbent layer (22), wherein said dressing (30) comprises at least one valved connector (26) connected to the sealing film (20), wherein the valved connector (26) is detachably connectable to at least one negative pressure generation and indication device (5).

In another embodiment, the sealing film (20) of the dressing is connected to at least one hose (23) equipped with at least one closed-system valved connector (26), wherein the valved connector (26) is detachably connectable to at least one negative pressure generation and indication device (5).

In one embodiment, the sealing film (20) comprises an impermeable or semi-permeable structure.

In one embodiment, the modular structure of the dressing allows different dressing configurations and sizes to be formed, according to medical need, wound size, body part shape, etc.

In one embodiment, the dressing (30) additionally comprises at least one colorimetric indicator (24) disposed in the internal environment of the dressing (30). In this embodiment, the formation of negative pressure in the dressing is done by a simple syringe.

In one embodiment, the colorimetric indicator (24) is positioned between the absorbent layer (22) and the sealing film (20) of the dressing, so that the colorimetric indicator (24) remains in direct contact with the air present within the dressing (30). In another alternative embodiment, the colorimetric indicator (24) is positioned within the closed-system valved connector (26) of the hose (23) of the dressing, so that the colorimetric indicator (24) also remains in direct contact with the air present within the dressing (30).

In one embodiment, the colorimetric indicator (24) comprises a pigment sensitive to changes in the concentration of CO₂ in the air, wherein the pigment is disposed on a surface and comprises at least one chemical compound that reacts and changes color when the concentration of CO₂ present inside the dressing is changed, i.e., when the user generates negative pressure (or vacuum) in the dressing from a negative pressure generation and indication device and changes the concentration of CO₂ in the dressing.

In one embodiment, the color change of the pigment may be related to a scale of negative pressure values to indicate a value or a range of negative pressure values according to the indicated color.

In one embodiment, the sensitive color change is detectable by means of algorithms and/or applications that relate the negative pressure values to the variation of red points of the RGB system detected in the color change of the paint, calculating and assigning the values or a range of negative pressure values to the detected color variations.

In this way, the dressing (30) with colorimetric indicator (24) for negative pressure therapy allows the detection of negative pressure within the dressing (30) at any time, allowing precise and easy monitoring of negative pressure by using just an application and/or algorithm for reading/scanning the color of the pigment and indicating the negative pressure.

Therefore, the dressing (30) with colorimetric indicator (24) allows negative pressure to be indicated using only a pigment arranged in the dressing itself (30), i.e., it does not use any mechanical or electronic components in the dressing (30), which provides greater comfort and flexibility in usability for the user, in addition to reducing manufacturing costs.

One embodiment of negative pressure therapy is a method for treating incisional wounds using negative pressure therapy comprising the steps of:
a. applying at least one dressing (30) over the wound on the user's skin (6), wherein the dressing (30) comprises an adhesive film (21) connected around a sealing film (20), wherein the sealing film (20) is positioned above at least one absorbent layer (22) and connected to at least one valved connector (26);
b. isolating the internal environment of the dressing (30) through adhesiveness of adhesive film (21) to the user's skin (6);
c. detachably connecting a negative pressure generation and indication device (5) to the valved connector (26);
d. generating negative pressure in the internal environment of the dressing (30) by means of the negative pressure generation and indication device (5);
e. indicating the range of negative pressure values to which the internal environment of the dressing (30) is subjected by means of the negative pressure generation and indication device (5); and
f. disconnecting the negative pressure generation and indication device (5) from the valved connector (26).

In one embodiment, the step of indicating the range of negative pressure values of the internal environment of the dressing (30) is performed by moving an indication mechanism (2) connected to the elastic element (4), which stretches and contracts along the length of a channel (13) of the negative pressure generation and indication device (5).

In this way, said therapeutic treatment is carried out with the aid of the previously defined negative pressure generation and indication device (5). In this sense, this allows negative pressure to be used for the treatment of incisional wounds, so that negative pressure is generated in the dressing and indicated by said device (5), and in which the device (5) is subsequently disconnected from the dressing, without the need for the device to remain coupled to the dressing.

In another embodiment, the negative pressure therapy of the invention is a cosmeceutical method for treating skin (6) comprising the steps of:
a. applying at least one dressing (30) to the user's skin (6), wherein the dressing (30) comprises an adhesive film (21) connected around a sealing film (20), wherein the sealing film (20) is positioned above at least one absorbent layer (22) and connected to at least one valved connector (26);
b. isolating the internal environment of the dressing (30) by means of adhesiveness of adhesive film (21) to the user's skin (6);
c. detachably connecting a negative pressure generation and indication device (5) to the valved connector (26);
d. generating negative pressure in the internal environment of the dressing (30) by means of the negative pressure generation and indication device (5);
e. indicating the range of negative pressure values to which the internal environment of the dressing (30) is subjected by means of the negative pressure generation and indication device (5); and
f. disconnecting the negative pressure generation and indication device (5) from the valved connector (26).

In one embodiment, the step of indicating the range of negative pressure values of the internal environment of the dressing (30) is performed by moving an indication mechanism (2) connected to the elastic element (4), which stretches and contracts along the length of a channel (13) of the second plunger (8) of the negative pressure generation and indication device (5).

In this way, said cosmeceutical treatment is carried out with the aid of the previously defined negative pressure generation and indication device (5). In this sense, this allows the negative pressure to be used for the treatment of skin (6) for cosmeceutical purposes, such as for the treatment of pimples, post-waxing treatment, pre-fight treatment in martial arts, treatment of pain in general, etc., so that the negative pressure is generated and indicated through said device (5), and wherein the device (5) is subsequently disconnected from the dressing, without the need for the device to remain attached to it.

The examples illustrated below are intended only to provide further elements to facilitate the materialization of some of the embodiments of the invention, and should not, however, be interpreted as limiting the invention.

### Example 1 - Negative pressure indicating syringe for use in negative pressure therapy dressing

In this Example, an indicating syringe (5) was developed with adapted and optimized geometry to be used as a negative pressure generator and indicator when connected to negative pressure therapy dressings. The indicator syringe (5) generates and indicates negative pressure mechanically, without the use of any electronic components.

The developed indicator syringe (5) has a geometry adapted to be connected to the dressing and disconnected when not in use, i.e., the user connects the syringe to the dressing, generates and/or checks the indication of negative pressure, and can then disconnect it.

In this way, the developed syringe (5) brings several benefits to the usability by the user and to the treatment using negative pressure therapy, since the indicator syringe (5) can be detached from the dressing when it is not being used, bringing greater comfort and practicality to the user in relation to prior art dressings that require a negative pressure generating device to remain connected to the dressing.

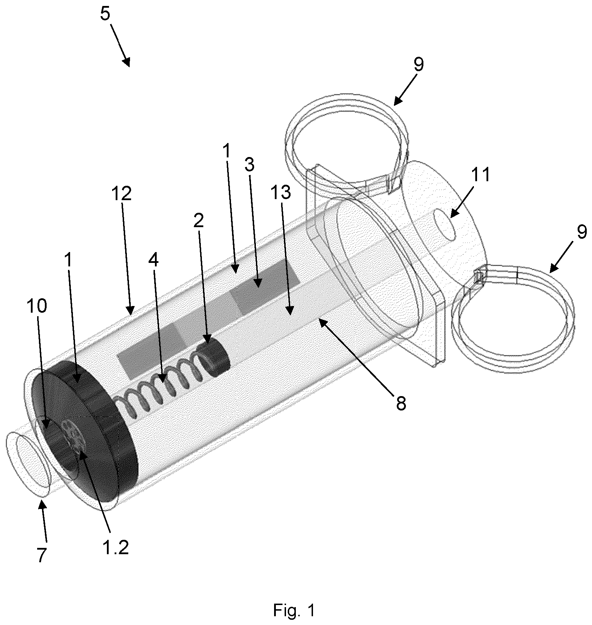

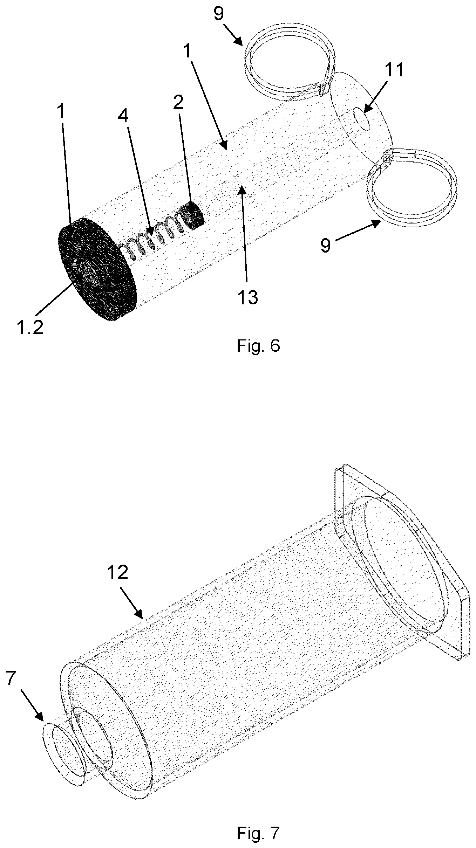

Specifically, the indicator syringe (5) has a body (12), as illustrated in Fig. 7, and a first plunger (1), as illustrated in Fig. 6, the first plunger (1) being positioned in a sliding manner in the inner part the body (12), just like the external shape of a common syringe. Figs. 1 to 5 show the syringe (5) formed by the first plunger (1) positioned in the inner part the body (12).

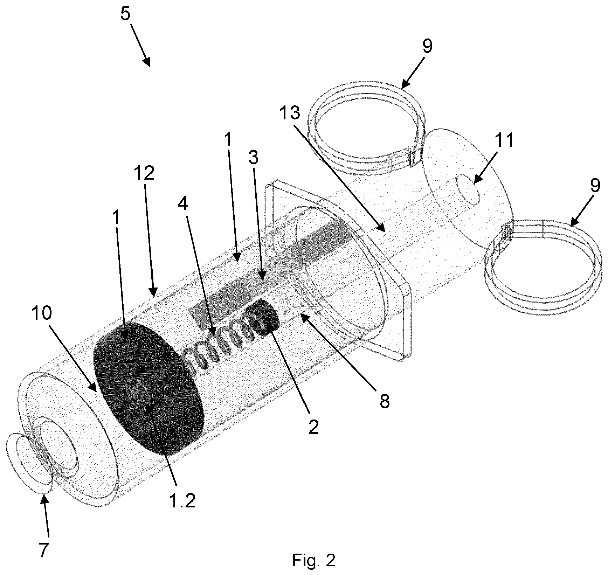

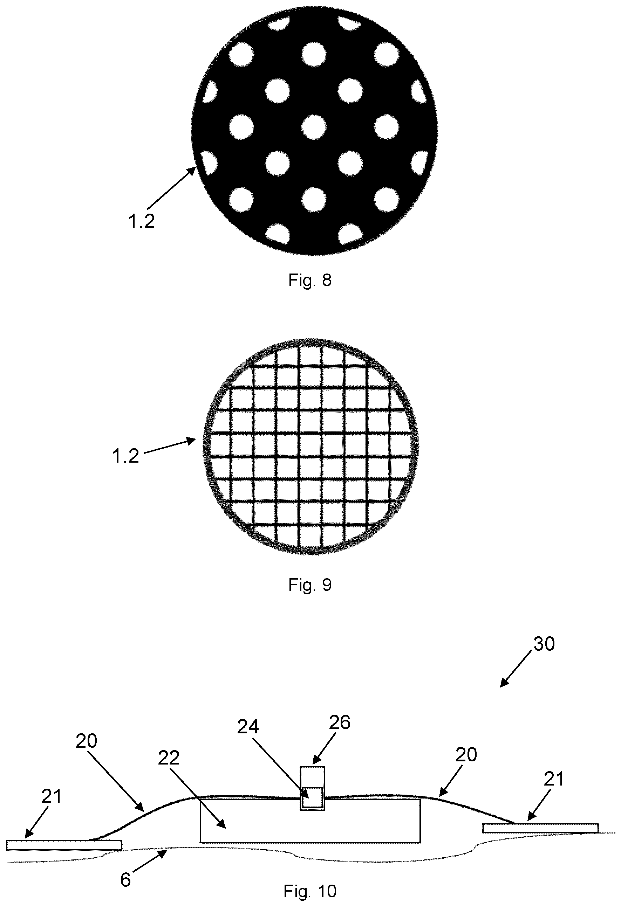

Figs. 1 and 2 show in detail the internal central region of the first plunger (1) that is hollowed out along the entire length of the first plunger (1), forming a second plunger (8) having an internal channel (13). In this internal channel (13), an indication mechanism (2) is arranged, connected to an elastic element, which in this Example is a spring (4), wherein the spring (4) is fixed to a flap (1.2) of the channel (13), so that the spring (4) stretches and contracts the indication mechanism (2) along the channel (13). The flap (1.2) is equipped with holes (hollowed out) to allow the passage of air coming from inside the dressing, as illustrated in figures 8 and 9.

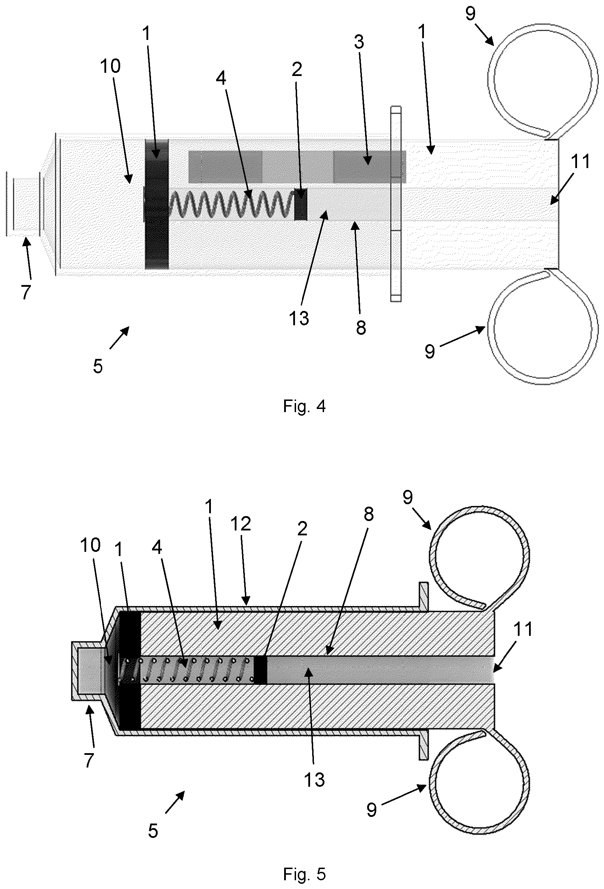

The indication mechanism (2) is a rubber that separates and isolates the channel (13) into two environments, a first environment with a hollow region for the intake of air from the internal environment of the dressing, and a second environment with a hollow region for the intake of atmospheric air. The indication mechanism (2) made of rubber is able to isolate the two environments so that there is no passage of air between the first environment and the second environment, and at the same time has adequate surface roughness that allows the free movement of extension and contraction of the spring (4), without restriction of movement due to friction. Fig. 5 shows a cross-sectional view of the developed indicator syringe (5), illustrating in detail the indication mechanism (2) separating the channel (13) into two environments, the first environment with a first end (10) with a flap (1.2) hollowed to receive air from the internal environment of the dressing, and the second environment with a second end (11) hollow to receive atmospheric air.

Furthermore, a visual indicator (3) is arranged parallel to the length of the channel (13), so that, as the indication mechanism (2) moves within the channel (13), the visual indicator (3) indicates the negative pressure range in which the internal environment of the dressing (30) is located.

In this Example, the visual indicator (3) is a green and red color band, where red indicates to the user that the negative pressure is in a range of values unsuitable for negative pressure therapy, and green indicates to the user that the negative pressure is in a range of values suitable for negative pressure therapy. For the purposes of this example, the optimal negative pressure range, i.e., the green band in the visual indicator (3) has values between -50 and -175 mmHg, and the unsuitable negative pressure range (the red band) has values between 0 and -50 mmHg and above -175 mmHg. More specifically, the optimal negative pressure range has values between -75 and -125 mmHg, and the inadequate negative pressure range has values between 0 and -75 mm Hg and above -125 mmHg. However, the adequate or optimal negative pressure values can be changed as advances in medicine, clinical trials, scientific research, etc. identify a better and more suitable optimal negative pressure range.

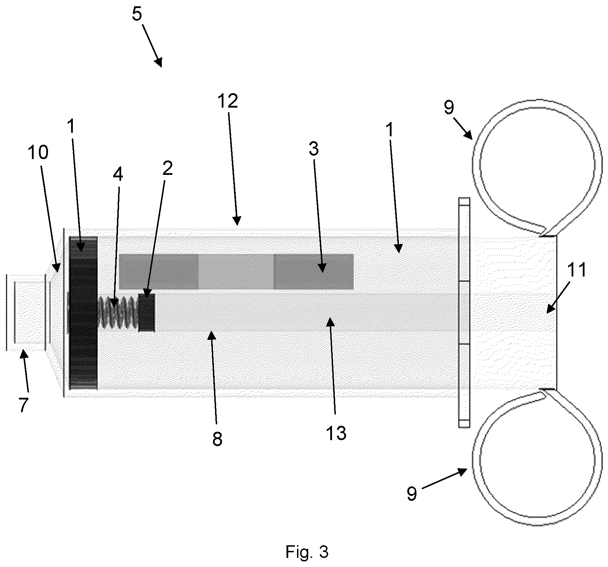

Fig. 3 shows a state in which the spring (4) is in a contracted position, which has moved the indication mechanism (2) into a position that indicates, by means of the visual indicator (3), that the pressure range detected in the internal environment of the dressing (30) is inadequate (red band) for treatment with negative pressure therapy.

Fig. 4 shows a state in which the first plunger (1) has been pulled and the spring (4) is in a compressed position, which has moved the indication mechanism (2) into a position that indicates, by means of the visual indicator (3), that the pressure range detected in the internal environment of the dressing (30) is adequate (green band) for treatment with negative pressure therapy.

Furthermore, the indicator syringe (5) is made of transparent material to allow visualization of the movement of the indication mechanism (2) within the plunger channel (1) and the indication of the pressure range by the visual indicator (3).

Furthermore, the first plunger (1) has a pulling handle (9) with a shape adapted to fit the user's fingers and assist him/her in the action of pulling the first plunger (1), i.e., in the action of generating negative pressure on the dressing. In this way, the handle (9) promotes greater accessibility/usability by allowing the user to pull the first plunger (1) using only one hand.

In this embodiment, the indicator syringe (5) has a Luer-Lock type nozzle (7), with threaded geometry for connection to a valved connector (26) connected to the negative pressure dressing.

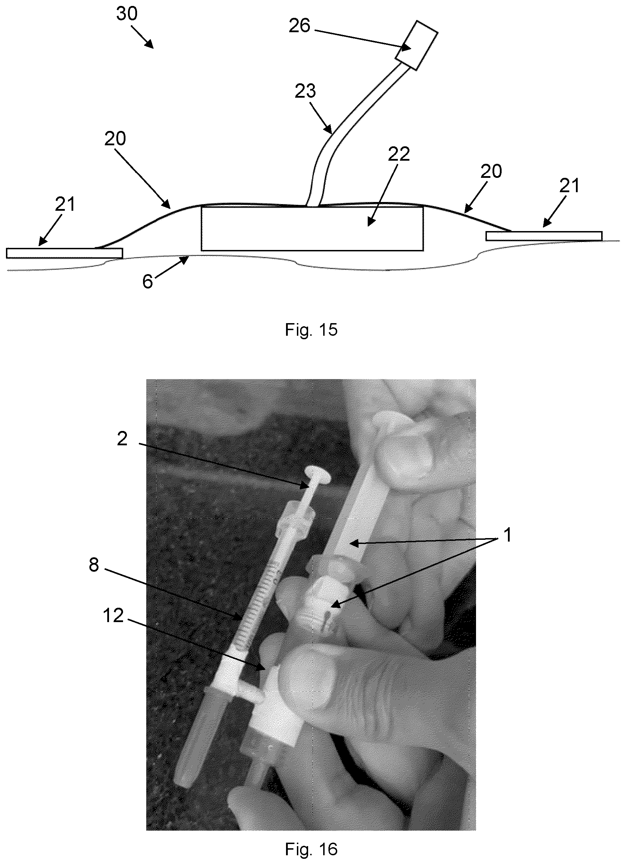

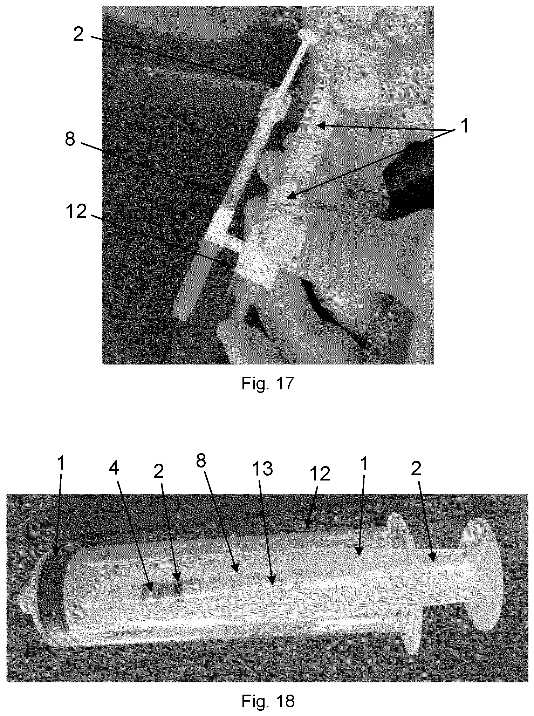

Figs. 16 and 17 show a test prototype of the indicator syringe (5) developed in this Example that illustrates how the syringe works to generate and indicate negative pressure. Fig. 16 illustrates the pulled first plunger (1) and the contraction movement of the indication mechanism (2) of the second plunger (8), and Fig. 17 illustrates the first plunger (1) inserted into the body (12) and the distension movement of the indication mechanism (2) of the second plunger (8). Fig. 18 shows another prototype of the developed indicator syringe (5), in which the second plunger (8) was arranged internally to the first plunger (1) and to the body (12), so as to be in communication with the air in the internal environment of the body (12) (i.e., the air coming from the internal environment of the dressing, when the indicator syringe (5) is connected to the dressing) and with the atmospheric air.

In this way, the developed indicator syringe (5) provides several benefits for both the user and the manufacturer, since it brings more comfort to the user by allowing the syringe to be disconnected from the dressing when not in use, as well as low manufacturing costs, since the syringe (5) has simple geometry and components that are easy to produce, with the possibility of the product being sold at a value much lower than the state-of-the-art negative pressure generation and indication devices.

Furthermore, the developed indicator syringe (5) is devoid of any electrical/electronic element, so that it operates without a battery or any form of electrical power supply, unlike prior art vacuum devices that use electronic and electrical components in automated vacuum generating devices. Therefore, the indicator syringe (5) of the present invention is a device that generates and indicates negative pressure in a purely mechanical manner.

Thus, the indicator syringe (5) contributes to the expansion of the use of negative pressure therapy to treat wounds, allowing more people to enjoy the benefits of negative pressure therapy.

Furthermore, the indicator syringe (5) developed in this Example can also be used to generate and indicate negative pressure in other dressings/devices that use vacuum for therapeutic treatments, such as, for example, in cupping therapy, which uses suction cups in the shape of "cups" to generate vacuum on the skin (6). Similar to the application of dressings, the indicator syringe (5) is connected to the vacuum valve of the suction cup, allowing the generation and indication of the negative pressure generated inside the suction cup, providing monitored cupping therapy.

### Example 2 - Dressing for use in negative pressure therapy

In this Example, a dressing (30) was developed for use in negative pressure therapy. The dressing (30) has a modular structure that allows the size and shape of the dressing to be adapted to any type and size of wound.

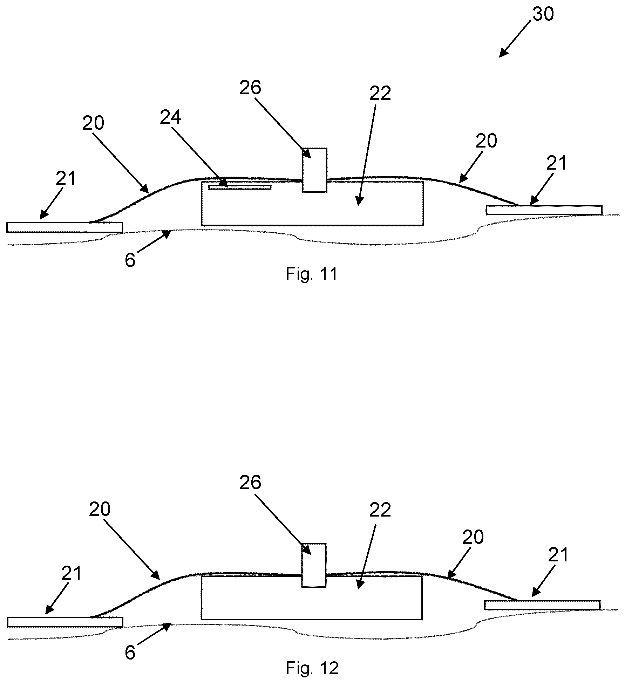

In a variation of format, the dressing (30) has a sealing film (20) connected with a connector (26), wherein the connector (26) is a closed-system valved connector, known in the art and commercially as Microclave^{™}, having a Luer-Lock nozzle that allows the detachable connection with a negative pressure generation and indication device (5). In this way, the connector (26) is embedded in the dressing (30), as illustrated in Fig. 12.

In another format variation, the dressing (30) has a sealing film (20) connected to a hose (23). The hose (23) has a closed-system valved connector (26) of the Microclave^{™} type with a Luer-Lock nozzle, which allows detachable connection to a negative pressure generation and indication device (5). In this case, the user can position the connector (26) in a more suitable way to connect to the device. Fig. 15 illustrates said variation described.

Furthermore, the hose (23) is made of flexible material, which does not cause discomfort to the user and allows the hose (23) to be moved in any direction, providing greater ergonomics for the correct positioning of the connector (26). In addition, the hose (23) has at least one fixing means that allows the hose (23) to be fixed to the dressing so that it does not become "loose" or "swinging" in the dressing, the fixing means being, for example, an adhesive face, a snap-on clip, or any detachable fixing means.

The sealing film (20) is made of impermeable or semi-permeable material.

An adhesive film (21) is arranged around the sealing film (20), wherein the adhesive film (21) seals and isolates the internal environment of the dressing from the air in the external environment, preventing atmospheric air from entering the dressing.

Below the sealing film (20) there is at least one absorbent layer (22), which comprises at least one of: non-stick polyurethane foam, gauze-type absorbent fabric with silver components and non-stick film; a combination thereof; or any absorbent material with or without non-stick film that prevents skin (6) damage associated or not with a medicinal or cosmetic compound.

In this embodiment, the sealing film (20) and the adhesive film (21) are manufactured from a material comprising a transparent plastic film.

Therefore, the dressing developed in this Example allows negative pressure to be indicated using only the negative pressure generation and indication device (5) developed in Example 1, so that the device (5) and the dressing (30) do not use any mechanical or electronic components, which provides greater comfort and flexibility in usability for the user, in addition to reducing manufacturing costs.

Furthermore, the developed dressing + generation and indication device are devoid of any electrical/electronic element, so that they operate without a battery or any form of electrical power supply, unlike the dressings + vacuum devices in the prior art that use electronic and electrical components in the automated vacuum generating devices.

### Example 3 - Dressing with colorimetric indicator for use in negative pressure therapy

In this Example, a dressing (30) for negative pressure therapy was developed having at least one colorimetric indicator (24). Said colorimetric indicator (24) is a dry ink/pigment arranged on a flexible thin tape/sheet, in which the ink was developed with chemical compounds that make it sensitive to variations in CO₂ concentration in the air.

In a variation of format, the dressing (30) has a valved connector (26) of the Microclave^{™} type, with a Luer-Lock nozzle that allows the detachable connection with a negative pressure generation and indication device (5). In this way, the connector (26) is embedded in the dressing (30).

In another format variation, the dressing (30) has a hose (23) connected to a closed-system valved connector (26) of the Microclave^{™} type with a Luer-Lock nozzle, which allows the detachable connection with a negative pressure generation and indication device (5).

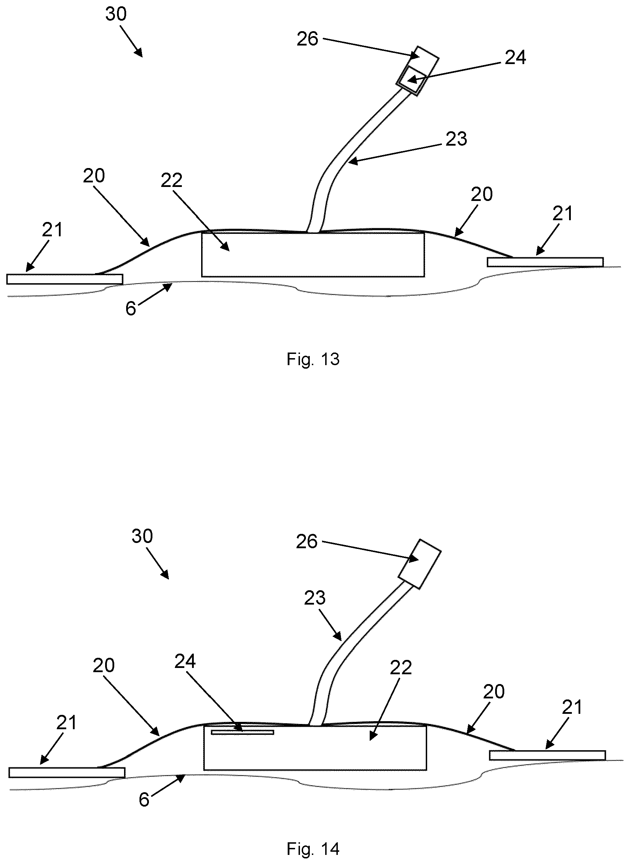

In one variant, the colorimetric indicator (24) is positioned between the absorbent layer (22) and the sealing film (20) of the dressing (30), so that the colorimetric indicator (24) remains in direct contact with the air present inside the dressing (30), as illustrated in figures 11 and 14.

In another variant, the colorimetric indicator (24) is positioned inside the closed-system valved connector (26) of the dressing (30), so that the colorimetric indicator (24) also remains in direct contact with the air present inside the dressing (30), as illustrated in figures 10 and 13.

In this sense, the ink of the colorimetric indicator (24) reacts and changes color when the concentration of CO₂ present inside the dressing (30) (or the connector (26)) is altered, i.e., when the user generates negative pressure (or vacuum) in the dressing (30) from a negative pressure generation and indication device and alters the concentration of CO₂ in the dressing (30).

The said colorimetric indicator (24) was developed based on the colorimetric indicator described in the scientific article (2020) "A general-purpose colorimetric air pressure indicator" authored by Dilidaer Yusufu and Andrew Mills, which describes two applications for the indicator, one to detect vacuum in vacuum packaging and the other as a pressure-sensitive ink in aerodynamics. Furthermore, the article mentions the use of the Thymol Blue (TB) pH indicator for vacuum detection, which is green at ambient pressure (1 atm), bright blue under high vacuum (< 0.01 atm) and bright yellow at high air pressures (> 10 atm).

In very small and sensitive negative pressure variations, as is the case of the negative pressure range used in negative pressure therapy, the colors of the ink of the developed colorimetric indicator (24) are proportional to a sensitive negative pressure value scale, so that the color change of the ink indicates a change in the negative pressure value.

In this way, the sensitive color change is detectable through algorithms and/or applications that relate the negative pressure values with the variation of red points of the RGB system detected in the color change of the paint, calculating and assigning the values or a range of negative pressure values to the color variations detected.

Thus, the dressing with colorimetric indicator (24) developed in this Example allows the detection of negative pressure within the dressing at any time, allowing precise and easy monitoring of negative pressure by using an application for reading/scanning the ink color and indicating negative pressure.

In this embodiment, the colorimetric indicator (24) developed in this Example can also be applied to other dressings/devices that use vacuum for therapeutic treatments, such as, for example, cupping therapy, which uses cup-shaped suction cups to generate vacuum on the skin (6). In this sense, the colorimetric indicator (24) is placed inside the suction cup, allowing the detection of the negative pressure generated inside the suction cup and providing monitored cupping therapy.

Furthermore, the colorimetric indicator ink (24) allows the indication of positive pressures, enabling the ink of the present invention to be applied in any controlled environments in which pressure monitoring is desired.

Therefore, the dressing with colorimetric indicator (24) developed in this Example allows negative pressure to be indicated using only a thin and flexible tape arranged on the dressing itself, i.e., it does not use any mechanical or electronic components in the dressing, which provides greater comfort and flexibility in usability for the user, in addition to reducing manufacturing costs.

Furthermore, the developed dressing is devoid of any electrical/electronic element, so that it works without a battery or any form of electrical power, unlike prior art vacuum devices that use electronic and electrical components in automated vacuum generating devices.

### Example 4 - Boot-shaped dressing for use in negative pressure therapy

In this Example, a boot-shaped dressing was developed, with geometry similar to the shape of an Unna Boot.

The boot developed is shaped to fit the foot and extend to the user's leg, so that the boot is sealed at the height of the leg and at the end of the feet before the toes, in order to isolate the internal environment of the boot and maintain negative pressure.

Furthermore, the boot has at least one sealing film (20) and at least one absorbent layer (22) arranged below the sealing film (20), i.e., the absorbent layer (22) is arranged between the sealing film (20) and the skin (6) of the user.

Furthermore, the absorbent layer (22) additionally or alternatively has at least one medicinal component on the skin (6) contact surface of the user.

Furthermore, the sealing film (20) is connected with at least one connector (26), wherein the connector (26) is a valved connector of the Microclave^{™} type with a Luer-Lock nozzle that allows detachable connection with a mechanical negative pressure generation and indication device (5). In another embodiment, the connector (26) is connectable with an electronic negative pressure generation and indication device in a non-detachable manner.

Furthermore, the developed boot performs negative pressure treatment with a suitable pressure range of -40 mmHg relative to atmospheric pressure.

In this way, when the boot connector (26) is connected to an electronic negative pressure generation and indication device, the negative pressure remains constant within a suitable pressure range inside the boot. And when the boot connector (26) is connected to a mechanical negative pressure generation and indication device (5), the negative pressure varies within a suitable predetermined range, depending on the use.

Those skilled in the art will appreciate the knowledge presented herein and will be able to reproduce the invention in the modalities presented and in other variants and alternatives, covered by the scope of the following claims.

## Claims

1. A dressing (30) for negative pressure therapy **characterized in that** it comprises:
- modular structure provided with an adhesive film (21) connected around a sealing film (20), wherein the sealing film (20) is positioned above at least one absorbent layer (22); and
- at least one valved connector (26) connected to the sealing film (20), wherein the valved connector (26) is detachably connectable to at least one negative pressure generation and indication device (5).

2. The dressing according to claim 1, **characterized in that** it additionally comprises a colorimetric indicator (24) of negative pressure positioned:
- between the absorbent layer (22) and the sealing film (20) of the dressing (30); or
- within the closed-system valved connector (26) of the dressing (30).

3. The dressing according to claim 1 or 2, **characterized in that** it comprises geometry in the shape of:
- a planar dressing for placing on the skin (6); or
- an Unna Boot, being sealed at the height of the leg and at the end of the foot before the toes.

4. A device for manual generation and indication of negative pressure in a dressing **characterized in that** it comprises at least two body-plunger sets:
- the first containing a body (12) detachably connectable to a dressing (30) to which negative pressure to be formed, and at least one first plunger (1) manually operable and arranged in a sliding manner in an internal region of the body (12); and
- the second containing a body fixedly connected to the first plunger (1) through at least one channel (13) communicating with the internal region of the body (12) and at least a second plunger (8) equipped with an indication mechanism (2) of negative pressure arranged internally to the channel (13),
the value of the negative pressure is indicated by means of at least one visual indicator (3) according to the movement of the indication mechanism (2) of the second plunger (8), caused by the manual activation of the first plunger (1).

5. The device according to claim 4, **characterized in that** it comprises at least one body (12) and at least one plunger (1) arranged in a sliding manner in an internal region of the body (12), and in which the device comprises:
- at least one channel (13) communicating with the internal region of the first plunger (1) that extends along the length of the first plunger (1);
- at least one indication mechanism (2) arranged internally to the channel (13); and
- at least one visual indicator (3) associated with the channel (13);
wherein the indication mechanism (2) moves along the inner part of the channel (13) to measure a negative pressure value, the value being indicated by means of the visual indicator (3).

6. The device according to claim 5, **characterized in that** said indication mechanism (2) comprises at least one elastic element (4), the elastic element comprising at least one of: sponge; polymer; spring; pneumatic spring; or a combination thereof.

7. The device according to claim 5, **characterized in that** said indication mechanism (2) is fixed to the elastic element (4), the elastic element (4) being fixed to a first end (10) of the inner part of the channel (13) by means of at least one flap (1.2), the elastic element (4) extending along the length of the channel (13).

8. The device according to claim 5, **characterized in that** said channel (13) of the second plunger (8) comprises two environments separated and isolated by the indication mechanism (2):
- a first environment with at least one first end (10) with at least one flap (1.2) hollow for air intake from the internal environment of the dressing; and
- a second environment with at least one second end (11) hollow for atmospheric air intake, in which the elastic element (4) stretches and contracts along the channel (13).

9. A process for manufacturing a negative pressure generation and indication device, **characterized in that** it comprises the following steps:
- forming a fluidic connection point on the body of a body-plunger set containing a body (12) removably connectable to a dressing (30) to which negative pressure to be formed, and at least one first plunger (1) manually operable and slidably arranged in an internal region of the body (12); and
- connecting said fluidic connection point to the body of a second body-plunger set through at least one channel (13) communicating with the internal region of the first body (12), the second body-plunger set comprising at least one second plunger (8) provided with an indication mechanism (2) of negative pressure arranged internally to the channel (13) of the second plunger (8).

10. The process according to claim 9, **characterized in that** it comprises the steps of:
- manufacturing of at least one body (12) comprising at least one nozzle with threaded geometry (7);
- manufacturing of at least one plunger (1) comprising the steps of:
a) opening an access to communicate at least one channel (13) to the internal region of the plunger (1), wherein the channel (13) extends along the length of the plunger (1);
b) arranging at least one indication mechanism (2) internally to the channel (13); and
c) arranging at least one visual indicator (3) associated with the channel (13);
- fitting the plunger (1) in a sliding manner into the internal region of the body (12).

11. The process according to claim 10, **characterized in that** the channel (13) opening is formed to comprise two environments separated and isolated by the indication mechanism (2):
- a first environment comprising at least a first end (10) with at least one flap (1.2) hollow for the intake of air from the internal environment of the dressing (30); and
- a second environment comprising at least a second end (11) hollow for the intake of atmospheric air.

12. A kit for negative pressure therapy, **characterized in that** it comprises:
- a dressing (30) comprising a modular structure provided with an adhesive film (21) connected around a sealing film (20) positioned above at least one absorbent layer (22) and at least one valved connector (26) connected to the sealing film (20), wherein the valved connector (26) is detachably connectable to at least one negative pressure generating device (5); and
- a device (5) comprising at least two body-plunger sets, the first containing a body (12) removably connectable to a dressing (30) to which negative pressure to be formed, and at least one first plunger (1) manually operable and slidably arranged in an internal region of the body (12), and
- the second containing a body fixedly connected to the body of the first plunger (1) through at least one channel (13) communicating with the internal region of the body (12) and at least a second plunger (8) equipped with an indication mechanism (2) of negative pressure arranged internally to the channel (13).

13. A negative pressure therapy, **characterized in that** it comprises:
- placing the dressing (30) of the invention on the skin (6);
- the manual generation of negative pressure in said dressing (30) until a negative pressure limit is obtained, indicated by a visual indicator (3); and
- one or more new steps for manually generating negative pressure when the negative pressure is close to or below a minimum negative pressure limit.
